Europäisches Patentamt

European Patent Office

Office européen des brevets

(19)

(11) EP 0 468 478 B1

(12) **EUROPEAN PATENT SPECIFICATION**

| | |
|---|---|
| (45) Date of publication and mention of the grant of the patent:<br>**02.10.1996  Bulletin 1996/40** | (51) Int Cl.6: **C12N 13/00, A61L 2/02** |

(21) Application number: **91112422.0**

(22) Date of filing: **24.07.1991**

(54) **Method for sterilizing an aqueous medium**

Verfahren zur Sterilisierung eines wässerigen Mediums

Procédé pour la stérilisation d'un milieu aqueux

(84) Designated Contracting States:
**CH DE FR GB LI NL SE**

(30) Priority: **26.07.1990 JP 198124/90**

(43) Date of publication of application:
**29.01.1992  Bulletin 1992/05**

(73) Proprietor: **House Food Industrial Co., Ltd.**
**Higashiosaka-shi Osaka-fu (JP)**

(72) Inventors:
• **Hattori, Ryuichi**
  **Kyoto-shi, Kyoto-fu (JP)**
• **Murao, Shigeru**
  **Kita-Katsuragi-gun, Nara-ken (JP)**

(74) Representative: **VOSSIUS & PARTNER**
**Postfach 86 07 67**
**81634 München (DE)**

(56) References cited:
FR-A- 1 039 422          FR-A- 2 421 145
GB-A- 947 699            US-A- 3 058 894
US-A- 4 663 292

• IEEE TRANSACTIONS ON INDUSTRY
  APPLICATIONS. vol. 24, no. 3, June 1988, NEW
  YORK US pages 387 - 394; MIZUNO, A. & HORI,
  Y.: 'Destruction of living cells by pulsed
  high-voltage application.'

EP 0 468 478 B1

**Description**

The present invention relates to a process for sterilizing an aqueous medium by applying high-voltage electric pulses.

More specifically, the present invention relates to a process for killing microorganisms contained in an aqueous medium by applying the discharge of high-voltage electric pulses.

Commonly used processes to kill microorganisms such as bacteria in an aqueous solution, are sterilization processes such as thermal sterilization or radiation sterilization. However, thermal sterilization processes generally need vast amounts of energy since the processes include a step of heating a solution to be treated at not less than 121°C under pressure to kill sporogenous bacteria such as Clostridium botulinum. The heating process may possibly cause denaturation of materials contained in the aqueous solution. The radiation sterilizaion processes require an effective shield for the leakage of radioactivity. Further concerns about possible contamination of food by radioactivity can not be disregarded.

In order to avoid the above problems, processes for sterilization comprising the discharge of high-voltage electric pulses have been proposed. By these processes, an aqueous solution can be easily sterilized without causing the denaturation of materials contained in the solution.

The Japanese Patent Unexamined Publication No. 61-239,880 discloses an example of a process for killing microorganisms in alcoholic beverages. The process comprises the step of discharging a high-voltage of from 10 to 70 KV in a liquor to generate a high-voltage plasma, by which microorganisms are killed. The Japanese Patent Unexamined Publication No. 61-239,946 discloses a process for sterilizing milk similar to the process described in the above patent document.

The Japanese Patent Unexamined Publication No. 63-82,666 discloses a process for killing microorganisms in water by applying a discharge of high-voltage electric pulses. The Japanese Patent Unexamined Publication No. 55-71,437 discloses a process for killing microorganisms comprising the step of applying a high electric field. Further, the Japanese Patent Unexamined Publication No. 62-151,174 discloses an apparatus for destroying cells by applying high-voltage electric pulses to a solution.

However, the application of the discharge of high-voltage electric pulses into solutions has been found to be insufficient for killing microorganisms. In particular, these processes were substantially noneffective against sporulating bacteria. Further, these processes which comprise the use of electric energy such as electric pulses, need a substantial amount of electric energy to entirely kill microorganisms, which is undesirable from an economical point of view.

An object of the present invention is to provide a process for sterilizing an aqueous medium which eliminates the problems mentioned above.

Another object of the present invention is to provide an efficient process for killing microorganisms in an aqueous medium without using a large amount of electric energy.

The inventors of the present invention have conducted various studies to achieve the foregoing objects and found that the degree of sterilization is remarkably increased by applying high-voltage electric pulses onto the surface of an aqueous medium rather than applying the pulses into the medium. The inventors also found that this process is particulary effective against sporulating bacteria which could not be killed by application of electric pulses into the medium.

Thus, in accordance with the above objects, the present invention provides a process for sterilizing an aqueous medium which comprises the step of applying the discharge of high-voltage electric pulses onto the surface of the medium containing microorganisms whereby the electrode does not touch the surface of the aqueous medium.

In accordance with another embodiment of the present invention, there is provided a process for sterilizing an aqueous medium which comprises the step of applying the discharge of high-voltage electric pulses under nitrogen onto the surface of the medium containing microorganisms.

The inventors of the present invention also found that these sterilizing processes became more effective for killing microorganisms when the process comprises the optional step of discontinuing the application of electric pulses for a period during which an optional ultrasonic irradiation is applied. The inventors further found that the denaturation of proteins or the acidification of the medium possibly caused by the application of electric pulses could be eliminated when the aqueous medium contained a sufficient amount of a buffer.

Thus, in accordance with yet another embodiment, the present invention provides a process for sterilizing an aqueous medium according to the process described above, wherein the step of applying the discharge of pulses further comprises the steps of:

(a) applying the discharge of high-voltage electric pulses onto the surface of an aqueous medium containing microorganisms;
(b) discontinuing the application of the discharge of pulses for a period during which ultrasonic irradiation may optionally be applied to the aqueous medium;
(c) resuming the application of the discharge of pulses after the discontinuation of the application of the discharge of pulses; and
(d) repeating steps (b) and (c), if desired.

In accordance with a still further embodiment, the present invention provides a process for sterilizing an aqueous medium according to the above-described process, wherein the aqueous medium comprises an effective amount of buffer sufficient to maintain the pH of the aqueous medium within the range of from 5 to 9 after the application of discharge of the pulses.

The invention will now be described with reference to the accompanying drawings.

FIG. 1 is a diagram of an electric circuit preferably used for the process of the present invention.

FIG. 2 is a drawing showing a waveform of the high-voltage electric pulse preferably used in the present invention.

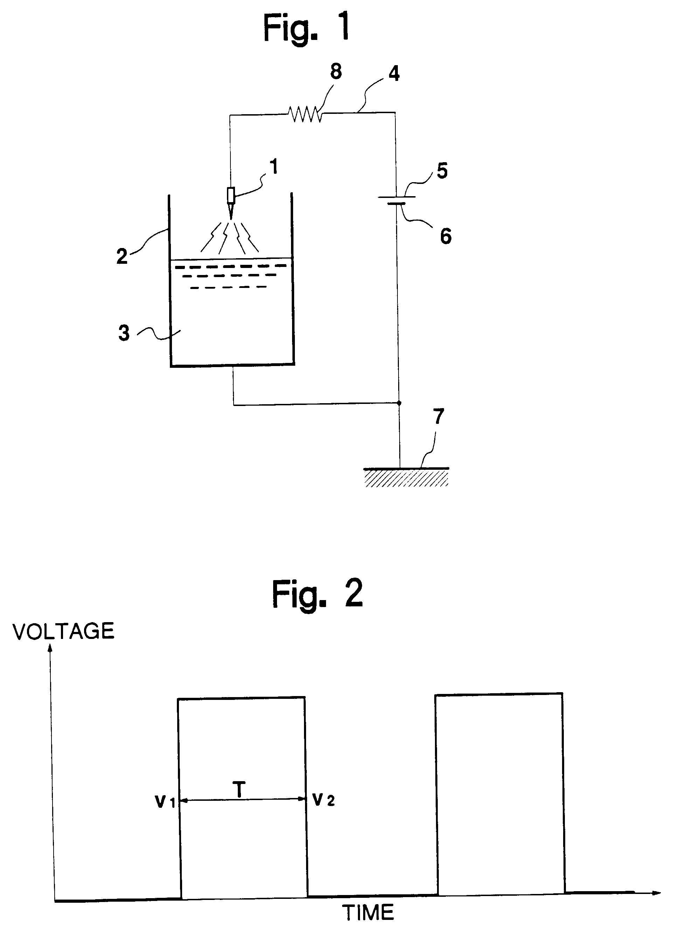

Referring now to FIG.1, there is shown an embodiment of the electric circuit preferably used for the process of the present invention, wherein the electrode 1, which is provided over the aqueous medium 3, is connected with the positive output of pulse generator 5 by way of the electric circuit 4 in which is provided the resistance 8. The bottom of container 2 is connected with the negative output of the pulse generator 6 by way of electric circuit 4, and the negative output of pulse generator 6 is grounded by way of earth 7.

Examples of the aqueous medium to be treated by the process of the present invention include drinking water such as, for example, utility water; water for industrial use such as, for example, water for manufacturing foods or pharmacueticals; water-based foods such as, for example, soup; and waste water such as, for example, waste water from medical institutions or laboratories. The temperature of the aqueous medium may generally be within the range of from 5 to 40°C for the application of the discharge of high-voltage electric pulses, however, the temperature is not limited to the above range.

Examples of the microorganisms, which may be contained in the aqueous medium to be sterilized by the process of the present invention, include bacteria such as, for example, sporulating bacteria or Escherichia coli; and fungi such as, for example, mold or yeast. The aqueous media to be treated by the present process may contain these microorganisms in an amount of from not more than $10^7$ cells/ml, preferably not more than $10^5$ cells/ml. By the application of the discharge of high electric pulses according to the present invention, most of the microorganisms in the aqueous medium will be physically or chemically damaged, i.e., partial or complete destruction or destruction of their internal cell structures, which will result in the death of the microorganisms.

The discharge of high-voltage electric pulse may be applied on the surface of the aqueous medium under constant conditions during the whole application, or the conditions of application of the discharge of pulses may be varied during the period of application, if desired. In general, the high-voltage electric pulse described hereinafter is preferably discharged under constant conditions.

The high-voltage electric pulse may be applied at any pulse-voltage that enable the pulse to be discharged aerially over the aqueous medium. If a pulse of higher voltage is used, the sterilization of the present process will become more effective, however, if the pulse-voltage is too high, the sterilization will be economically disadvantageous because of a high-performance pulse generator required. Thus, the pulse-voltage may preferably be within the range of from 5 to 50 KV, more preferably 20 KV, when the distance between the electrode and the surface of the aqueous medium is about 5 mm.

FIG. 2 shows a non-limiting example of the waveform of the high-voltage electric pulse. The waveform of the high-voltage electric pulse is not limited to the square wave shown in FIG. 2, but pulses in various kinds of waveform such as, for example, bow wave, or sine wave may be used in the present invention. Among these electric pulses, an electric pulse in square waveform is preferably used.

Referring to the waveform illustrated in FIG. 2, the pulse width of the high-voltage electric pulse is defined to be the time T from the time of the half value of the maximum rising pulse voltage ($V_1$) to the time of half value of the maximum falling pulse voltage ($V_2$). The sterilization will become more effective if a pulse of wider width is applied, however, if the width of the electric pulse is too wide, the process will be accompanied with disadvantages such as, for example, electrolysis or an excess of exothermic heat. Thus, preferably used high-voltage electric pulses are those having pulse widths within the range of from 0.01 to 1,000 μs, more preferably 1 μs.

Referring to the waveform illustrated in FIG. 2, the pulse frequency of the high-voltage electric pulse is defined to be the number of times per second that the voltage proceeds from the maximum pulse voltage at the end of the rising to the maximum pulse voltage at the end of next rising of pulse. The sterilization will become more effective if the pulse of higher frequency is discharged. However, if the frequency of the pulse is too high, a constant discharge path will not be obtained, which will result in an insufficient discharge on the surface of the aqueous medium. Thus, preferably used high-voltage electric pulses are those having pulse frequencies within the range of from $10^1$ to $10^5$, more preferably $10^3$. The pulse sequence of the high-voltage electric pulse may be a sequence such as, for example, a periodic pulse sequence, random pulse sequence, or Poisson pulse sequence.

The electrode used for the discharging of high-voltage electric pulses on the aqueous medium may be one of various kinds of electrodes which are fully or partly made of conductive materials such as, for example, tungsten, stainless steel, platinum, or graphite. The electrode may be in a shape of, for example, a needle, a sheet, a sphere, or a line.

The electrode may be provided over the aqueous medium so that the length of aerial discharge path from the surface of the aqueous medium to the electrode can be not more than 30 mm, preferably about 5 mm. If the electrode touches the surface of the aqueous medium or is entirely or partly immersed in the aqueous medium, the sterilizing effect on microorganisms, particulary on sporulating bacteria, will become significantly reduced. The electrode may be provided at any position over the aqueous medium as long as the high-voltage electric pulse can be discharged on the surface of the aqueous medium.

The period for discharging the high-voltage electric pulses may be increased or decreased depending on the type or the amount of microorganism contained in the aqueous medium, the discharging conditions of the high-voltage electric pulses, the kind of dissolved substances contained in the aqueous medium, or the temperature of the aqueous medium to be treated.

The process of the present invention is characterized in that the high-voltage electric pulses are aerialy, i.e., discharged on the surface of the aqueous medium to kill microorganisms, as described hereinbefore.

According to one embodiment of the present invention, the high-voltage electric pulses may be discharged in a nitrogen gaseous atmosphere. The nitrogen gas may be pure nitrogen gas or nitrogen gas containing a small amount of air, oxygen gas, or argon gas. The nitrogen gaseous atmosphere may generally be formed by introducing a flow of nitrogen gas into the headspace of the container containing the aqueous medium.

According to another embodiment of the present invention, the process for sterilization of the aqueous medium described above may further comprise the steps of:

(a) discharging high-voltage electric pulses onto the surface of the aqueous medium containing microorganisms;
(b) discontinuing the discharge of high-voltage electric pulses for a period of time during which ultrasonic irradiation may optionally be applied to the aqueous medium;
(c) resuming the discharge of high-voltage electric pulses after step (b); and
(d) repeating steps (b) and (c), if desired. According to this preferred embodiment, the sterilization of the aqueous medium can be conducted more effectively and economically.

In step (b), the discontinuation of the discharge of electric pulses may be continued for a period of time after the discharging step (a). The period of time may be increased or decreased depending on the conditions of sterilization such as, for example, the type of microorganism to be killed, the kind of dissolved substances contained in the aqueous medium such as, for example, sodium chloride, or the temperature of the aqueous medium.

For the discharge of high-voltage electric pulses in step (c), which follows the step (b) of discontinuing the discharge of pulses, the high-voltage electric pulses having the same characteristics as those used in the preceding discharging process (a) may be used. Alternatively, high-voltage electric pulses having different pulse-width or pulse-frequency may be used, if desired.

Ultrasonic waves, used in the ultrasonic irradiation optionally carried out in step (b), may be those having a frequency of not less than 16 KHz, preferably not less than 20 KHz. For the application of the ultrasonic irradiation to the aqueous medium during the period of discontinuation of the pulses, any type of ultrasonic generator which can generate the above-defined ultrasonic waves may be used.

The conditions for the application of ultrasonic irradiation can be varied widely and are not restrictive. For example, where 0.1 ml of the aqueous medium is treated with ultrasonic waves of 20 KHz frequency using an ultrasonic gererator having the maximum output of 200 W, the ultrasonic irradiation may be applied for three minutes at an output of 100 W/hr. Where the high-voltage electric pulse is discharged for a long period of time, the ultrasonic irradiation may preferably be applied for a long period of time. The ultrasonic irradiation may be continued during the whole of the period of discontinuating the discharge, or applied during a part of the period of step (b). The discharge of high-voltage electric pulses may preferably be resumed soon after the application of ultrasonic irradiation.

The step of discharging electric pulses (step A) and the step of discontinuing the discharge (step B) may optionally be repeated in the sterilization process, by means of which an excess of exothermic heat by the discharge of pulses can be reduced and the increase of temperature of the aqueous medium can be eliminated.

The pH of the aqueous medium is sometimes lowered by the discharge of the high-voltage electric pulses on the surface of the aqueous medium, which may result in the reduction of the sterilizing effect, the denaturation of protein, or the increase of acidity because of the alteration of the properties of the aqueous medium. In order to prevent the occurrence of these disadvantages, the sterilization process may preferably be applied to the aqueous medium comprising an effective amount of buffer by which the pH of the aqueous medium can be maintained within the range of from 5 to 9 after the discharge of the electric pulses. If a sufficient amount of buffer is originally contained in the aqueous medium by which the pH of the aqueous medium can be maintained within the range of from 5 to 9 after the discharge of the electric pulses, it will not be necessary to add additional amount of buffer to the aqueous medium. Generally, however, a necessary amount of buffer is preferably added before starting the discharge of the electric pulses.

Examples of the aqueous medium originally containing a sufficient amount of buffer include, for example, soups such as, for example, corn soup, potage soup, or consomme soup; and sauces such as, for example, white sauce or sauce demiglace. Although these aqueous media are sufficiently buffered, an additional amount of buffer may further be added to these aqueous medium before applying the discharge of electric pulses.

The buffer may be any type of material having buffering action in an aqueous solution, which should not be considered limiting. Non-limiting examples of the buffer include, for example, a mixture of monobasic potassium phosphate and dibasic sodium phosphate, a mixture of monobasic potassium phosphate and sodium borate, and trisaminomethane dissolved in an aqueous hydrochloric acid. These buffers may be used in combination.

Where a buffer is added to the aqueous medium, a buffer in the form of a powder or solid may be added directly to the aqueous medium, or alternatively, the buffer may be dissolved in an aqueous solvent such as water to prepare a concentrated aqueous buffer solution to be added to the aqueous medium.

The buffer may be added to the aqueous medium in a amount sufficient to maintain the pH of the aqueous medium within the range of from 5 to 9 at the end of the discharge of the electric pulses. If the discharge of pulses is continued for a longer period or a pulse of wider pulse-width is used, the acidification of the aqueous medium will be significant. In these cases, it will be required to add a substantial amount of buffer to prevent the occurrence of these disadvantages.

Where the discharge of the high-voltage electric pulse is applied for 20 minutes using the electric pulse shown in FIG. 2, which has a pulse-voltage of 20 KV and the pulse frequency of 1,000 Hz, the pH of ion-exchange water (20 ml) will become 3 or less soon after the beginning of the application of pulses and the pH of the medium will be lowered gradually during the discharging process. If the discharge is applied under the same conditions described above to the ion-exchange water containing 1/15 M phosphate buffer, the pH of the aqueous solution will be maintained at not less than 7 after 20 minutes, by which time effective sterilization can be attained.

For example, it is preferred to add a phosphate buffer containing a mixture of $6.12 \times 10^{-5}$M of acidic buffer such as monobasic potassium phosphate and $1.00 \times 10^{-3}$M of basic buffer such as dibasic sodium phosphate to the aqueous medium having the pH of 8.04, before the discharge of the pulses in order to prevent the pH of the aqueous medium from going below 5.0 after the discharge of pulses for ten minutes.

Referring to another example, it is preferred to add a buffer containing a mixture of $7.05 \times 10^{-2}$M of said acidic buffer and $2.08 \times 10^{-3}$M of said basic buffer to the aqueous medium having a pH of 5.29, before the discharge of pulses in order to prevent the pH of the aqueous medium from going below 5.0 after the discharge of pulses for ten minutes.

The aqueous medium treated by the process of the present invention may be further treated by a thermal sterilization or other known sterilization processes. For example, a low temperature thermal sterilization of the aqueous medium at a temperature of not more than 100°C after treatment by the process of the present invention can attain an effective sterilization, which is quite useful from a practical point of view.

The process of the present invention is useful since the process ensures a considerable and economical killing of microorganisms in the aqueous medium, and the denaturation or decomposition of ingredients contained in the aqueous medium can be eliminated. If compared with a sterilization process comprising the discharge of electric pulses directly into an aqueous medium, a shorter period of time of discharging pulses, i.e, a smaller amount of electric energy, is required by the process of the present invention to obtain the same degree of sterilization as that obtained by the comparative process. Further, the present process is useful for killing sporulating bacteria which are almost resistant to the process comprising the discharge of electric pulses directly into an aqueous medium.

The present invention will be further illustrated by the following Examples. The Examples are given by way of illustration only and are not to be construed as limiting.

Example 1

The bacterial strain <u>Bacillus subtilis</u> ATCC 6633 was cultured on a standard agar medium at 35 °C for 7 days. The cultured bacteria were treated with thermal sterilization at 90 °C for 20 minutes to kill the vegetative cells, and the resulting bacteria were suspended in ion-exchange water in the amount of $10^5$ cells/ml to prepare the aqueous medium containing microorganisms.

In the electric circuit shown in FIG. 1, 100 ml of the aqueous medium prepared above was filled in the container 2 whose bottom was made of stainless steel, and the electrode made of stainless steel in a shape of a needle was provided over the aqueous medium at a position 5 mm from the surface of the aqueous medium. The electrode 1 was connected with the positive output of pulse 5 by way of the electric circuit 4 in which resistance 8 is provided. The bottom of container 2 was connected with the negative output of pulse 6 by way of electric circuit 4, and the negative output of pulse 6 was grounded by way of ground 7.

The discharge of high-voltage electric pulses having the waveform shown in FIG.2 was applied to the surface of the aqueous medium continuously for 15 minutes under the following conditons without using resistance 8: pulse voltage: 20 KV; polarity of output: positive; pulse width: 1 µs; and pulse frequency: 1,000 Hz.

Example 2

The sterilization of the aqueous medium was carried out for 30 minutes using resistance of 13.32 KΩ in the same manner as described in Example 1, with the exception that the bacterial strain Bacillus subtilis ATCC 6633 was cultured on a standard agar medium at 35°C for 7 days, and the cultured bacteria were treated with thermal sterilization at 80°C for 20 minutes to kill the mycelium and the cells were then suspended in ion-exchange water in the amount of $10^7$ cells/ml to prepare the aqueous medium.

Comparative Example 1

The sterilization of the aqueous medium was carried out in the same manner as described in Example 2, with the exception that the stainless electrode in the shape of a needle was immersed in the aqueous medium to a position 5 mm below the surface of the medium, and the discharge of the pulses was applied for 40 minutes.

Example 3

The bacterial strain Escherichia coli ATCC 11775 was cultured on a standard agar medium at 35 °C for 2 days. The cultured bacteria were suspended in 20 ml of ion-exchange water in the amount of $10^7$ cells/ml to prepare the aqueous medium containing microorganisms.

In the electric circuit shown in FIG. 1, 20 ml of the aqueous medium prepared above was filled in the container 2 whose bottom was made of stainless steel. The electrode 1 made of stainless steel in a shape of a needle was provided over the aqueous medium at a position 5 mm from the surface of the aqueous medium.

The discharge of high-voltage electric pulses having the waveform shown in FIG.2 was applied to the surface of the aqueous medium continuously for 30 seconds under the following conditons without using resistance 8: pulse voltage: 20 KV; polarity of output: positive; pulse width: 1 μs; and pulse frequency: 1,000 Hz.

Example 4

The sterilization of the aqueous medium was carried out in the same manner as described in Example 3, with the exception that the discharge of the high-voltage electric pulses was applied continuously for 60 seconds.

Comparative Example 2

The sterilization of the aqueous medium was carried out in the same manner as described in Example 3, with the exception that the stainless electrode in the shape of a needle was immersed in the aqueous medium to a position 5 mm below the surface of the medium, and the discharge of the pulses was applied for 10 minutes.

Comparative Example 3

The sterilization of the aqueous medium was carried out in the same manner as described in Comparative Example 2, with the exception that the discharge of the pulses was applied for 15 minutes.

From the results summarized in the following Table 1, it is clearly understood that the process of the present invention is effective against sporulable bacteria, i.e., Bacillus subtilis, as well as Escherichia coli. The sterilization effect was calculated by the following formula:

$$\text{Sterilizing effect} = \text{Log} \frac{(\text{number of bacteria after discharging})}{(\text{number of bacteria before discharging})}$$

Table 1

| Example No. | Sterilizing effect |
|---|---|
| Example 1 | -2.54 |
| Example 2 | -4.55 |
| Comparative Example 1 | -0.03 |
| Example 3 | -4.30 |
| Example 4 | -5.26 |
| Comparative Example 2 | -3.73 |
| Comparative Example 3 | -4.17 |

Example 5

The bacterial strain <u>Bacillus subtilis</u> ATCC 6633 was cultured on a standard agar medium at 35 °C for 7 days. The cultured bacteria were treated with thermal sterilization at 90 °C for 20 minutes to kill the vegetative cells, and the resulting bacteria were suspended in 100 ml of ion-exchange water in the amount of $10^5$ cells/ml to prepare the aqueous medium containing microorganisms.

In the electric circuit shown in FIG. 1, 100 ml of the aqueous medium prepared above was filled in the container 2 whose bottom was made of stainless steel, and the electrode made of stainless steel in a shape of a needle was provided over the aqueous medium at a position 5 mm from the surface of the aqueous medium. The discharge of high-voltage electric pulses having the waveform shown in FIG.2 was applied to the surface of the aqueous medium continuously for 10 minutes, which was followed by the discontinuation of the discharge for 10 minutes, and then the discharge of pulses was applied for 6 minutes. The electric pulses of the following characteristics were applied without using resistance 8: pulse voltage: 20 KV; polarity of output: positive; pulse width: 1 µs; and pulse frequency: 1,000 Hz.

Example 6

The sterilization of the aqueous medium was carried out in the same manner as described in Example 5, with the exception that the electric pulses were applied for 5 minutes, followed by a discontinuation of discharge for 15 minutes, a resumption of the application of pulses for 5 minutes, a discontinuation of discharge for 15 minutes, and finally, an application of the electric pulses for 6 minutes.

Example 7

The sterilization of the aqueous medium was carried out in the same manner as described in Example 5, with the exception that the electric pulses were applied for 14 minutes, followed by a discontinuation of discharge for 10 minutes, and then a resumption of the electric pulses for 2 minutes.

Example 8

The sterilization of the aqueous medium was carried out in the same manner as described in Example 5, with the exception that the electric pulses were applied for 10 minutes, followed by a discontinuation of discharge for 30 minutes, and then a resumption of the electric pulses for 6 minutes.

Example 9

The sterilization of the aqueous medium was carried out in the same manner as described in Example 5, with the exception that the electric pulses were applied for 8 minutes, followed by a discontinuation of discharge for 10 minutes, and then a resumption of the electric pulses for 8 minutes.

Example 10

The sterilization of the aqueous medium was carried out in the same manner as described in Example 5, with the exception that the electric pulses were applied for 6 minutes, followed by a discontinuation of discharge for 5 minutes, and then a resumption of the electric pulses for 10 minutes.

From the results summarized in the following Table 2, it should be apparent to one ordinary skilled in the art that the process for sterilization of the present invention is quite effective while consuming only small amount of electrical energy.

Table 2

| Example No. | Sterilizing effect |
|---|---|
| Example 5 | -4.13 |
| Example 6 | -4.13 |
| Example 7 | -3.58 |
| Example 8 | -3.22 |
| Example 9 | -3.82 |
| Example 10 | -3.71 |

Example 11

The bacterial strain Bacillus subtilis ATCC 6633 was cultured on a standard agar medium at 35 °C for 7 days. The cultured bacteria were treated with thermal sterilization at 80 °C for 20 minutes to kill the vegetative cells, and the resulting bacteria were suspended in 100 ml of a buffer solution (pH 7.0). The buffer solution was obtained by mixing 1/15 M monobasic potassium phosphate solution and 1/15 M dibasic sodium phosphate solution in the ratio of 3 : 7. The cells were suspended in an amount to prepare the aqueous medium containing $10^6$ cells/ml of microorganisms.

In the electric circuit shown in FIG. 1, 100 ml of the aqueous medium (pH 7.0) prepared above were filled in the container 2 whose bottom was made of stainless steel, and the electrode made of stainless steel in a shape of a needle was provided over the aqueous medium at a position 5 mm from the surface of the aqueous medium. The discharge of high-voltage electric pulses having the waveform shown in FIG.2 was applied to the surface of the aqueous medium continuously for 15 minutes, followed by a discontinuation of the discharge for 3 minutes, and then a resumption of the discharge of pulses for 15 minutes. The electric pulses of the following characteristics were applied without using resistance 8: pulse voltage: 20 KV; polarity of output: positive; pulse width: 1 μs; and pulse frequency: 1,000 Hz.

During the discontinuation of the discharge of electric pulses, ultrasonic irradiation of a frequency of 20 KHz was applied to the aqueous medium using an ultrasonic generator (Tomy Seiko, Co., Ltd., UR-200P) at the output of 100 W/hr for 3 minutes.

Example 12

The sterilization of the aqueous medium was carried out in the same manner as described in Example 11, with the exception that the electric pulses were applied for 20 minutes, followed by a discontinuation of discharge for 3 minutes, and then a resumption of the electric pulses for 10 minutes.

Example 13

The sterilization of the aqueous medium was carried out in the same manner as described in Example 12, with the exception that the cultured bacteria were suspended in 100 ml of ion-exchange water in the amount of $10^6$ cells/ml.

Example 14

The bacterial strain Escherichia coli ATCC 11775 was cultured on a standard agar medium at 35 °C for 2 days. The cultured bacteria were suspended in 20 ml of ion-exchange water in an amount of $10^7$ cells/ml to prepare the aqueous medium containing microorganisms.

In the electric circuit shown in FIG. 1, 20 ml of the aqueous medium prepared above was filled in the container 2 whose bottom was made of stainless steel, and the electrode 1 made of stainless steel in a shape of a needle was provided over the aqueous medium at a position 5 mm from the surface of the aqueous medium. The discharge of high-voltage electric pulses having the waveform shown in FIG.2 was applied to the surface of the aqueous medium continuously for 15 seconds, which was followed by a discontinuation of the discharge for 10 minutes, and then the discharge of pulses was applied for 15 seconds. The electric pulses of the following conditions were applied without using resistance 8: pulse voltage: 20 KV; polarity of output: positive; pulse width: 1 μs; and pulse frequency: 1,000 Hz.

Example 15

The sterilization of the aqueous medium was carried out in the same manner as described in Example 14, with the exception that the electric pulses were applied for 30 seconds, followed by a discontinuation of discharge for 10 minutes, and then the resumption of the electric pulses for 30 seconds.

From the results summarized in the following Table 3, it should be apparent to one ordinary skilled in the art that the process of the present invention comprising the application of ultrasonic irradiation is quite effective only with a small amount of electric energy. It is also apparent that the present process is effective against Escherichia coli.

Table 3

| Example No. | Sterilizing effect |
|---|---|
| Example 11 | -4.37 |
| Example 12 | -4.33 |
| Example 13 | -2.80 |

Table 3   (continued)

| Example No. | Sterilizing effect |
|---|---|
| Example 14 | -5.38 |
| Example 15 | -6.26 |

Example 16

The bacterial strain <u>Bacillus subtilis</u> ATCC 6633 was cultured on a standard agar medium at 35 °C for 7 days. The cultured bacteria were treated with thermal sterilization at 80 °C for 20 minutes to kill the vegetative cells, and the resulting bacteria were suspended in 100 ml of a buffer solution (pH 7.0). The buffer soluticn was obtained by mixing 1/15 M monobasic potassium phosphate solution and 1/15 M dibasic sodium phosphate solution in the ratio of 3 : 7. The cells were suspended in an amount to prepare an aqueous medium containing $10^5$ cells/ml of microorganisms.

In the electric circuit shown in FIG. 1, 100 ml of the aqueous medium (pH 7.0) prepared above was filled in the container 2 whose bottom was made of stainless steel, and the electrode made of stainless steel in a shape of a needle was provided over the aqueous medium at a position 5 mm from the surface of the aqueous medium. The discharge of high-voltage electric pulses having the waveform shown in FIG.2 was applied to the surface of the aqueous medium continuously for 30 minutes. The electric pulses of the following characteristics were applied without using resistance 8: pulse voltage: 20 KV; polarity of output: positive; pulse width: 1 µs; and pulse frequency: 1,000 Hz.

Example 17

The sterilization of the aqueous medium was carried out in the same manner as described in Example 16, with the exception that the electric pulses were applied for 25 minutes, followed by a discontinuation of discharge for 5 minutes, and then a resumption of the electric pulses for 5 minutes.

Example 18

The bacterial strain <u>Bacillus subtilis</u> ATCC 6633 was cultured on a standard agar medium at 35 °C for 7 days. The cultured bacteria were treated with thermal sterilization at 80 °C for 20 minutes to kill the vegetative cells, and the resulting bacteria were suspended in 100 ml of ion-exchange water in the amount of $10^6$ cells/ml to prepare the aqueous medium containing microorganisms.

In the electric circuit shown in FIG. 1, 30 ml of the aqueous medium prepared above was filled in the container 2 whose bottom was made of stainless steel, and an electrode made of stainless steel in a shape of a needle was provided over the aqueous medium at a position 5 mm from the surface of the aqueous medium. The headspace of the container was filled with nitrogen gas by introducing a flow of nitrogen gas (nitrogen content of 99.99%) at a rate of from 3.0 to 3.5 liter/minute, and then the discharge of high-voltage electric pulses having the waveform shown in FIG.2 was applied to the surface of the aqueous medium continuously for 20 minutes. Electric pulses of the following characteristics were applied without using resistance 8: pulse voltage: 20 KV; polarity of output: positive; pulse width: 1 µs; and pulse frequency: 1,000 Hz.

Example 19

The sterilization of the aqueous medium was carried out in the same manner as described in Example 18, with the exception that the electric pulses were applied for 10 minutes, followed by a discontinuation of discharge for 15 minutes, and then a resumption of the electric pulses for 10 minutes.

Example 20

The sterilization of the aqueous medium was carried out in the same manner as described in Example 18, with the exception that the cultured bacteria were suspended in a buffer solution (pH 7.0) obtained by mixing 1/15 M monobasic potassium phosphate solution and 1/15 M dibasic sodium phosphate solution in the ratio of 3 : 7, and the pulses were applied at the pulse voltage of 15 KV.

Example 21

The sterilization of the aqueous medium was carried out in the same manner as described in Example 19, with the exception that ultrasonic irradiation at the frequency of 28 KHz was applied to the aqueous medium using an ultrasonic generator (Tomy Seiko, Co., Ltd., UR-20P) at a output of 20 W/hr during the discontinuation of the discharge of electric pulses.

The results obtained above are summarized in the following Table 4.

Table 4

| Example No. | pH of the medium (before discharge) | pH of the medium (after discharge) | Sterilizing effect |
|---|---|---|---|
| Example 16 | 7.15 | 7.04 | -3.34 |
| Example 17 | 7.15 | 7.04 | -3.55 |
| Example 18 | - | - | -5.60 |
| Example 19 | - | - | -5.56 |
| Example 20 | 7.15 | 7.08 | -3.13 |
| Example 21 | - | - | -6.56 |

## Claims

1. A process for sterilizing an aqueous medium containing a microorganism which comprises the step of applying a discharge of high-voltage electric pulses onto the surface of the medium whereby the electrode does not touch the surface of the aqueous medium.

2. The process for sterilizing an aqueous medium according to claim 1, wherein the aqueous medium comprises an effective amount of buffer sufficient to maintain the pH of the aqueous medium within the range of from 5 to 9 after the application of the discharge of electric pulses.

3. The process for sterilizing an aqueous medium according to claim 1 or 2,wherein the step of applying is carried out under a nitrogen atmosphere.

4. The process for sterilizing an aqueous medium according to claim 1, wherein the microorganism is a sporulating bacteria.

5. The process for sterilizing an aqueous medium according to any one of claims 1 to 4, wherein the step of applying the discharge of high-voltage electric pulses further comprises the steps of:

   (a) applying the discharge of high-voltage electric pulses onto the surface of the aqueous medium;
   (b) discontinuing the application of the discharge;
   (c) resuming the application of the discharge after step (b); and
   (d) repeating the steps (b) and (c), if necessary.

6. The process for sterilizing an aqueous medium according to any one of claims 1 to 5, wherein ultrasonic irradiation is applied to the aqueous medium during at least a part of the discontinuation of step (b).

## Patentansprüche

1. Verfahren zur Sterilisierung eines wäßrigen Mediums, das einen Mikroorganismus enthält, das das Anlegen einer Entladung von Hochspannungsstromstößen an die Oberfläche des Mediums umfaßt, wobei die Elektrode die Oberfläche des wäßrigen Mediums nicht berührt.

2. Verfahren zur Sterilisierung eines wäßrigen Mediums nach Anspruch 1, bei dem das wäßrige Medium eine wirksame Menge an Puffer enthält, die zur Aufrechterhaltung des pH-Werts des wäßrigen Mediums innerhalb des Bereichs von 5 bis 9 nach dem Anlegen der Entladung von Stromstößen ausreicht.

3. Verfahren zur Sterilisierung eines wäßrigen Mediums nach Anspruch 1 oder 2, bei dem das Anlegen unter einer Stickstoffatmosphäre erfolgt.

4. Verfahren zur Sterilisierung eines wäßrigen Mediums nach Anspruch 1, bei dem der Mikroorganismus ein sporu-

**EP 0 468 478 B1**

lierendes Bakterium ist.

5. Verfahren zur Sterilisierung eines wäßrigen Mediums nach einem der Ansprüche 1 bis 4, bei dem das Anlegen der Entladung von Hochspannungsstromstößen weiterhin folgende Schritte umfaßt:

(a) Anlegen der Entladung von Hochspannungsstromstößen an die Oberfläche des wäßrigen Mediums;
(b) Unterbrechen des Anlegens der Entladung;
(c) Fortsetzen des Anlegens der Entladung nach Schritt (b); und
(d) Wiederholen der Schritte (b) und (c), falls erforderlich.

6. Verfahren zur Sterilisierung eines wäßrigen Mediums nach einem der Ansprüche 1 bis 5, bei dem zumindest während eines Teils der Unterbrechung von Schritt (b) das wäßrige Medium mit Ultraschall behandelt wird.

**Revendications**

1. Procédé de stérilisation d'un milieu aqueux contenant un micro-organisme, qui comprend une étape consistant à appliquer une décharge d'impulsions électriques à haute tension sur la surface du milieu, l'électrode ne touchant pas la surface du milieu aqueux.

2. Procédé de stérilisation d'un milieu aqueux selon la revendication 1, où le milieu aqueux comprend une quantité de tampon suffisante pour maintenir le pH du milieu aqueux dans la plage de 5 à 9 après l'application d'une décharge d'impulsions électriques.

3. Procédé de stérilisation d'un milieu aqueux selon la revendication 1 ou 2, où l'étape d'application s'effectue sous une atmosphère d'azote.

4. Procédé de stérilisation d'un milieu aqueux selon la revendication 1, où les micro-organismes sont des bactéries sporulantes.

5. Procédé de stérilisation d'un milieu aqueux selon l'une quelconque des revendications 1 à 4, où l'application de la décharge d'impulsions électriques à haute tension comprend les étapes consistant à :

(a) appliquer la décharge d'impulsions électriques à haute tension sur la surface du milieu aqueux :
(b) interrompre l'application de la décharge;
(c) reprendre l'application de la décharge après l'étape (b); et
(d) répéter les étapes (b) et (c), si nécessaire.

6. Procédé de stérilisation d'un milieu aqueux selon l'une quelconque des revendications 1 à 5, où on applique un traitement par ultrasons au milieu aqueux durant au moins une partie de l'interruption de l'étape (b).

# Fig. 1

# Fig. 2